# NEUE EUROPÄISCHE PATENTSCHRIFT

(11) **EP 0 347 776 B2**
(45) Veröffentlichungstag und Bekanntmachung des Hinweises auf die Entscheidung über den Einspruch: **10.07.2002**
(45) Hinweis auf die Patenterteilung: 26.08.1992
(21) Anmeldenummer: 89110996.9
(22) Anmeldetag: 16.06.1989
(51) Int. Cl.: A61K 6/06

(54) **Dispergierte Keramikmasse**
Dispersed ceramic material
Masse céramique dispersée

(30) Priorität: 21.06.1988 DE 3820839
(43) Veröffentlichungstag der Anmeldung: 27.12.1989
(73) Patentinhaber: Vita Zahnfabrik H. Rauter GmbH & Co. KG, D-79713 Bad Säckingen (DE)
(72) Erfinder: Thiel, Norbert, Dr., D-7880 Bad Säckingen (DE)
(74) Vertreter: Werner, Hans-Karsten, Dr.Dipl.-Chem.

(56) Entgegenhaltungen:
- EP-A- 0 185 597
- EP-A- 0 280 985
- DD-A- 263 406
- DE-A- 2 534 504
- DE-B- 1 296 301
- DE-C- 292 406

## Beschreibung

Gegenstand der vorliegenden Erfindung ist eine trockene Keramikmasse zur Herstellung von Keramikzähnen, -brücken und -kronen im zahntechnischen Labor, bestehend aus sinterfähigem Keramikpulver.

Derartige Keramikmassen sind bisher im zahntechnischen Labor hergestellt worden aus den handelsüblichen sinterfähigen Keramikpulvern und destilliertem Wasser oder mit den Keramikpulvern gemeinsam angebotenen Modellierflüssigkeiten. Aus der DE-OS 36 38 065 und der US-PS 4,645,454 ist auch schon bekannt, als dispergierende Flüssigkeit relativ hoch siedende organische Lösungsmittel einzusetzen, deren Brechungsindex in der Nähe des Brechungsindexes des Keramikpulvers liegt. Hierdurch ist es möglich, schon bei der Herstellung im ungebrannten Zustand beurteilen zu können, wie die Keramikmasse im gebrannten Zustand aussehen wird. Einige der dort vorgeschlagenen organischen Lösungsmittel sind bereits so viskos, daß der Keramikbrei zu zähflüssig wird. Es muß daher gegebenenfalls ein Gemisch von Lösungsmitteln angewendet werden, um den Viskositätskoeffizienten kleiner als 20 mPa·s zu halten. In der US-PS 4,645,454 ist weiterhin erwähnt, daß es prinzipiell möglich ist, das organische Lösungsmittel und das Keramikpulver vorher zu mischen und als vorgefertigte Mischung abgepackt auf den Markt zu bringen. Bisher sind aber derartige Gemische noch nicht auf den Markt gebracht worden. Ein wesentlicher Nachteil der bisher vorgeschlagenen organischen Lösungsmittel ist deren starke Geruchsbelästigung beim Arbeiten, die relativ hohe Toxizität, da Zahntechnikerdazu neigen, den Pinsel in den Mund zu nehmen, sowie die starke Geruchsbelästigung im Labor beim anschließenden Ausbrennen der Massen im Sinterofen, was ohne Abzug im Zahnlabor nicht mehr möglich wäre.

Die DE-B-12 96 301 beschreibt ein Verfahren zur Herstellung von künstlichen Zähnen, bei dem eine feuerfeste Oxidmasse mit einem hohen Anteil hochfeuerfester Bestandteile zwei Stunden lang bei einer Temperaturzwischen 1.300 und 1.750°C unter Sinterung und Rekristallisieren des Oxides, jedoch ohne wesentliches Schmelzen, gebrannt wird.

Die DE-C-292 406 beschreibt ein Verfahren zur Herstellung von künstlichen Zähnen durch den Zahnarzt ohne jegliche Angabe der verwendeten keramischen Masse. Erwähnt wird lediglich, daß der Schwindungskoeffizient des Zahnes beim Brennen geändert wird und sich durch mehr oder weniger Zusatz,ein größerer oder kleinerer Zahn erzielen läßt.

Aus der DE-OS 25 34 504 ist ein keramisches Material bekannt, welches zur Herstellung einer polykristallinen, gesinterten Keramik bzw. Sinterkeramik geeignet ist, wobei Calciumionen mit Phosphationen in wäßrigem Medium bei einem pH-Wert von 10 bis 12 gefällt werden und diese Ausfällung bei Temperaturen von mindestens 1000°C gesintert werden. Zur Erhöhung der Porosität wird dem Präzipitat ein organisches Bindemittel wie Cellulose oder Collagen beigemischt, welches sich bei den Temperaturen des Brennens vollständig verflüchtigt. Diese Materialien sind ungeeignet zur Herstellung von Keramikzähnen, -brücken und -kronen im zahntechnischen Labor, da hierbei im allgemeinen nur Temperaturen zwischen 930 und 960°C zur Anwendung kommen. Das gleiche gilt für die Massepulver zur Herstellung von handelsüblichen künstlichen Zähnen, die ebenfalls im allgemeinen bei ca. 1200°C während eines Zeitraums von 15 bis 20 Minuten gebrannt werden. Sofern also derartigen Massepulvern organische Farbstoffe oder organische Bindemittel wie Stärke oder Öl zugegeben werden, können sie bei den angewandten Temperaturen wesentlich leichter verbrannt werden.

Das gleiche gilt für poröse Keramik- oder Metallüberzüge und -erzeugnisse gemäß DE-OS 26 20 694, wo sogar bei Temperaturen von 1600°C gearbeitet wird. Im zahntechnischen Labor wird hingegegen meist nur 2 Minuten bei Temperaturen von 930 bis 960°C gearbeitet, so daß damit gerechnet werden muß, daß hochpolymere organische Substanzen nicht oder nur unvollständig verbrennen.

Die vorliegende Erfindung hat sich die Aufgabe gestellt, vorgefertigte Keramikmassen zur Herstellung von Keramikzähnen, -brücken und -kronen im zahntechnischen Labor zu entwickeln, die aus sinterfähigem Keramikpulver bestehen, jedoch nicht die oben aufgezeigten Nachteile aufweisen. Vorgefertigte Keramikmassen besitzen gegenüber den bisher üblichen sinterfähigen Keramikpulvern, die stets erst von Hand mit destilliertem Wasser oder den oben genannten organischen Lösungsmitteln dispergiert werden mußten, den Vorteil, daß keine Fehler durch falsche Mischungsverhältnisse entstehen und die Zeit für das Dispergieren eingespart werden kann. Es wurde jetzt gefunden, daß diese Aufgabe überraschend einfach gelöst werden kann durch eine Keramikmasse gemäß Patentanspruch.

Es war überraschend, daß die einsetzbaren Verdickungsmittel rasch und ohne störende Rückstände schon beim Brennen innerhalb von 2 Minuten bei 930 bis 960°C entfernt werden können, ohne daß dabei die mechanischen oder optischen Eigenschaften des gebrannten Materials beeinträchtigt werden. Obwohl bekannt ist, daß die verwendeten Verdickungsmittel relativ hochmolekular sind und beim Verbrennen dazu neigen, zunächst zu verkohlen und dabei Ruß zu bilden, wurde jetzt gefunden, daß sie als Beimengung zur dispergierten Keramikmasse für zahntechnische Labors beim anschließenden Brennen praktisch rückstandsfrei entfernt werden können. Ein weiterer Vorteil ist, daß sie bei diesem Brennen praktisch geruchsfrei verbrennen, so daß es auch weiterhin möglich ist, im zahntechnischen Labor Brennöfen ohne Abzug einzusetzen. Die verwendeten Verdickungsmittel sind zum anderen in der Lage, einzeln oder als Gemisch eingesetzt zu werden und dabei das verwendete Lösungsmittel so zu binden und die Viskosität so zu regulieren, daß dispergierte Keramikmassen entstehen, die sich - auch längere Zeit an der Luft ausgesetzt - gut verarbeiten lassen. Erst beim anschließenden Trocknen und Brennen bei höheren Temperaturen werden dann das Lösungsmittel und das Verdickungsmittel entfernt bzw. verbrannt. Ein weiterer Vorteil der Verdickungsmittel ist, daß sie gewünschtenfalls vom Zahntechniker mit etwas mehr Lösungsmitteln versetzt werden können, so daß sich der Zahntechniker die optimale Viskosität und Verarbeitbarkeit des Materials individuell einstellen kann.

Die dispergierende Flüssigkeit für die Keramikmasse enthält 0,02 bis 3 Gew.-% des Verdickungsmittels. Die optimale Menge ergibt sich unter anderem auch daraus, wieviel dispergierende Flüssigkeit, bezogen auf die Menge des sinterfähigen Keramikpulvers, eingesetzt werden soll. Der Gehalt an dispergierender Flüssigkeit in den fertigen Keramikmassen beträgt 15 bis 30 Gew.-%, da sich derartige Gemische besonders gut mit dem Spatel oder Pinsel verarbeiten lassen.

Den dispergierten Keramikmassen können gewünschtenfalls modifizierende Zusätze beigemischt werden, wie Weichmacher und grenzflächenaktive Substanzen. Weitere derartige Zusätze sind beispielsweise Stabilisierungsmittel, die das Wachstum von Bakterien und Pilzen verhindern. Als weitere Zusätze kommen Glykole in Frage, mit deren Hilfe es möglich ist, die Konsistenz, Modellierbarkeit, Standfestigkeit, den Feuchtigkeitsverlust und die Haltbarkeit in weiten Grenzen zu variieren.

Die neuen dispergierten Keramikmassen lassen sich in ähnlicher Weise mit dem Spatel oder Pinsel verarbeiten wie die heute ebenfalls üblichen Kunststoffmassen. Es ist daher auch möglich, die Arbeiten von Personal ausführen zu lassen, welches mit der Verarbeitung von Kunststoffmassen vertraut ist. Dies bedeutet eine weitere Einsparung von Zeit und Kosten aus Keramikpulver und einer Lösung der Verdickungsmittel erhalten wurden. Werden das trockene Keramikpulver und die trockenen Verdickungsmittel nur trocken miteinander vermischt und werden diese Gemische mit dem Lösungsmittel angerührt, erhält man im aligemeinen nur sehr schlecht verarbeitbare dispergierte Keramikmassen.

Diese durch Eintrocknen erhaltenen erfindungsgemäßen trockenen Gemische aus Keramikmasse und Verdickungsmittel lassen sich schnell, leicht und zuverlässig mit Wasser, niederen Alkoholen und/oder anderen leicht flüchtigen organischen Lösungsmitteln anrühren und wieder zur dispergierten Keramikmasse umwandeln. Es hat sich gezeigt, daß die so erhaltenen dispergierten Keramikmassen in den Eigenschaften wieder den ursprünglichen dispergierten Keramikmassen entsprechen. Sie lassen sich vom Zahntechniker wesentlich leichter, einfacher und zuverlässiger anrühren als reine Keramikmassen mit einem Lösungsmittel ohne die erfindungsgemäß verwendeten Verdickungsmittel.

Ein technisch bedeutungsvoller Vorteil dieser erfindungsgemäßen eingetrockneten Keramikmassen mit Verdickungsmittel ist, daß sie leichter abpackbar, lagerfähig und transportfähig sind als fertig dispergierte Keramikmassen. Diese eingetrockneten Keramikmassen mit Verdickungsmittel müssen zwar wie früher die reinen Keramikmassen vor der Benutzung mit dem Lösungsmittel angerührt werden. Durch den Zusatz von Verdikkungsmitteln ist dies jedoch wesentlich einfacher möglich, da die Menge des Lösungsmittels weniger kritisch ist als bei reinen Keramikpulvern und den zugehörigen Modellierflüssigkeiten. Ein weiterer erheblicher Vorteil sind die verbesserten plastischen Eigenschaften dieser Massen und damit die Möglichkeit der Anwendung der sogenannten Spateltechnik, die sonst im wesentlichen auf dem Dentalkunststoffsektor angewandt wird.

In den nachfolgenden Beispielen sind einige Ausführungsformen der trockenen Keramikmassen näher erläutert.

### Beispiel 1

96 g eines handelsüblichen sinterfähigen Keramikpulvers der Anmelderin (VMK 68 N - Dentin 252) wurden mit 12 g einer 1,5 %-igen wäßrigen Lösung von Xanthangummiderivat (Rhodegel 23/Fa. Rhone-Poulence) und 12 g einer 2%-igen wäßrigen Lösung von Hydroxypropyl-methylcellulose (Methocel F 4 M/Fa. Dow Chemical) und 8 g destilliertem Wasser mittels eines Kneters zu einer gut streichfähigen Paste angeteigt. Die entstandene Masse wurde auf eine große Fläche ausgebreitet und bei Raumtemperatur innerhalb von 48 Stunden getrocknet. Anschließend wurde die Masse mit einem Mörser zu einem Pulver verrührt. Dieses Pulver konnte mit destilliertem Wasser in kurzer Zeit wieder zu einer Paste der ursprünglichen Konsistenz angemischt werden.

Zum Vergleich wurden 96 g des gleichen Keramikpulvers mit 0,1 g Xanthangummiderivat und 0,24 g Hydroxypropyl-methylcellulose trocken vermischt. Beim Versuch, dieses trockene Gemisch mit der entsprechenden Menge destilliertem Wasser anzurühren, wurden unbrauchbare Produkte erhalten. Auch ein Vermahlen der Cellulose, anschließendes Absieben und trockenes Untermischen der entsprechenden feineren Körnung führte nicht zum Erfolg.

### Beispiel 2

12 g eines handelsüblichen sinterfähigen Keramikpulvers der Anmelderin (VMK 68 N - Dentin 552) wurden mit 3 g einer 1,5 %-igen wäßrigen Lösung von Hydroxybutylcellulose (Methocel HB/Fa. Dow Chemical) und 1 g destilliertem Wasser mit einem Achat- oder Glasspatel auf einer Glasplatte angemischt. Die Masse wurde anschließend in einer Porzellanschale 4 Stunden in einem Trockenofen mit Umluft bei 80°C getrocknet. Anschließend wurde die Masse in einem Mörser zu Pulver verrührt. Dieses Pulver konnte mit destilliertem Wasser rasch zu einer gut verarbeitbaren Paste angemischt werden.

Zum Vergleich wurde die gleiche Menge des Keramikpulvers mit 0,045 g Hydroxybutylcellulose intensiv trocken vermischt. Beim Versuch, dieses Gemisch mit Wasser anzurühren, wurden unbrauchbare Produkte erhalten.

### Beispiel 3

Die getrockneten Pulver gemäß Beispiel 1 und Beispiel 2 wurden anstatt mit destilliertem Wasser mit einer Mischung aus 95 % destilliertem Wasser und 5 % Butandiol angerührt. Auch hierbei wurden rasch gut verarbeitbare Pasten erhalten, die nach dem Trocken und Brennen einwandfreie Produkte lieferten.

## Patentansprüche

1. Trockene Keramikmasse bestehend aus sinterfähigem Keramikpulver und einem oder mehreren Verdickungsmitteln, erhältlich durch Eintrocknen einer Dispersion von Keramikpulver und einer Lösung von 0,2 bis 3 Gew.-% eines oder mehrerer Verdickungsmittel aus der Gruppe Zellulose, Zellulosederivate, Traganth, Xanthangummi und Alginate in Wasser, niederen Alkoholen und/oder anderen leicht flüchtigen organischen Lösungsmitteln, wobei gewünschtenfalls modifizierende Zusätze beigemischt sind und der Gehalt an Flüssigkeit 15 bis 30 Gew.-% der Dispersion des Keramikpulvers beträgt, wobei anschließend mit einem Mörser zu einem Pulver verrührt ist.

## Claims

1. A dry ceramic composition consisting of a sinterable ceramic powder and one or more thickening agents, obtainable by drying up a dispersion of ceramic powder and a solution of from 0.2 to 3% by weight of one or more thickening agents selected from the group consisting of cellulose, cellulose derivatives, tragacanth, xanthan gum and alginates in water, lower alcohols and/or other volatile organic solvents; wherein modifying additives may be admixed if desired, and the content of liquid is from 15 to 30% by weight of the dispersion of the ceramic powder, followed by trituration with a mortar to form a powder.

## Revendications

1. Masse céramique sèche composée d'une poudre céramique frittable et d'un ou plusieurs épaississants, que l'on peut obtenir en asséchant une dispersion de poudre céramique et une solution de 0,2 à 3 % en poids d'un ou plusieurs épaississants du groupe comprenant la cellulose, des dérivés cellulosiques, l'adragante, la gomme de xanthane et des alginates, dans de l'eau, des alcools inférieurs et/ou d'autres solvants organiques facilement volatils, à laquelle sont ajoutés éventuellement des additifs modifiants, la teneur en liquide étant de 15 à 30 % en poids de la dispersion de la poudre céramique, la masse étant ensuite broyée en une poudre dans un mortier.
